# EUROPÄISCHE PATENTANMELDUNG

(11) **EP 2 036 898 A2**
(43) Veröffentlichungstag der Anmeldung: **18.03.2009**
(21) Anmeldenummer: 07116371.1
(22) Anmeldetag: 13.09.2007
(51) Int. Cl.: C07D 317/34, C07D 317/72, C07D 409/14, C07D 491/10, C07D 451/10, C07D 451/12, A61K 31/438, A61P 11/00

(54) **Verfahren zur Herstellung von 1,3-Dioxolan-2Onen sowie von Carbonsäureestern durch Transacylierung unter Basischen Reaktionsbedingungen**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Die Erfindung betrifft ein Verfahren zur Herstellung von 1,3-Dioxolan-2-onen der allgemeinen Formel **3** 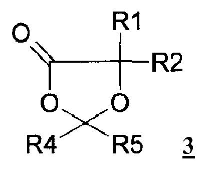 unter basischen Reaktionsbedingungen durch Umesterung des entsprechenden Esters der allgemeinen Formel **1**, 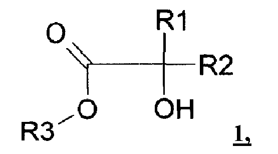 worin R₁ bis R₅ die in den Ansprüchen und der Beschreibung genannten Bedeutungen haben. Die Erfindung betrifft ferner ein Verfahren zur Herstellung von 2-Hydroxycarbonsäureestern der allgemeinen Formel **5** 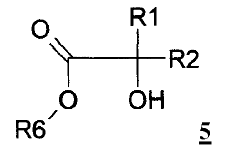 ohne oder mit Isolierung der Zwischenstufe in Form eines Derivats des 1,3-Dioxolan-2-ons der allgemeinen Formel **3** unter basischen Reaktionsbedingungen durch Umesterung des entsprechenden Esters der allgemeinen Formel **1**, worin R₁, R₂ und R₆ die in den Ansprüchen und der Beschreibung genannten Bedeutungen haben. Das erfindungsgemäße Verfahren erlaubt eine Umsetzung unter sehr schonenden basischen Bedingungen, die im Vergleich zu Reaktionen in hochpolaren aprotischen Lösemitteln weniger Nebenreaktionen und höhere Ausbeute bereitstellt. Eine Synthese von säure-, und/oder temperaturempfindlichen Verbindungen ist möglich.

## Beschreibung

### Technisches Gebiet der Erfindung

Die Erfindung betrifft ein Verfahren zur Herstellung von 1,3-Dioxolan-2-onen der allgemeinen Formel **3** 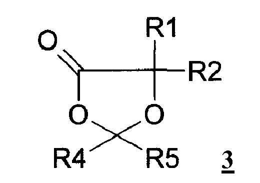 unter basischen Reaktionsbedingungen durch Umesterung des entsprechenden Esters der allgemeinen Formel **1,** 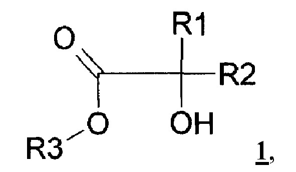 worin R₁ bis R₅ die in den Ansprüchen und der Beschreibung genannten Bedeutungen haben. Die Erfindung betrifft ferner ein Verfahren zur Herstellung von 2-Hydroxycarbonsäureestem der allgemeinen Formel **5** 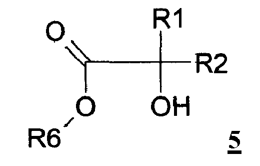 ohne oder mit Isolierung der Zwischenstufe in Form eines Derivats des 1,3-Dioxolan-2-ons der allgemeinen Formel **3** unter basischen Reaktionsbedingungen durch Umesterung des entsprechenden Esters der allgemeinen Formel **1,** worin R₁, R₂ und R₆ die in den Ansprüchen und der Beschreibung genannten Bedeutungen haben.

Das erfindungsgemäße Verfahren erlaubt eine Umsetzung unter sehr schonenden basischen Bedingungen, die im Vergleich zu Reaktionen in hochpolaren aprotischen Lösemitteln weniger Nebenreaktionen und höhere Ausbeute bereitstellt. Eine Synthese von säure-, und/oder temperaturempfindlichen Verbindungen ist möglich.
Die Kombination Zeolith Typ 4a und tert.-Alkoholat katalysiert Umesterungsreaktionen mit Scopin ohne dass nennenswert Umlagerungen zum Scopolin stattfinden.

### Hintergrund der Erfindung

Bei Acetoniden oder Derivaten des 1,3-Dioxolans handelt es sich um cyclische Ketale , die beispielsweise aus 1,2-Diolen, Ketonen oder Aldehyden hergestellt werden. Im Stand der Technik ist bekannt, dass saure Katalysatoren diese Reaktion unterstützen und/oder ermöglichen. Die cyclischen Ketale stellen synthetisch wertvolle Verbindungen dar, die aufgrund ihrer besonderen Eigenschaften nutzbringend anwendbar sind.

Beim speziellen Fall der 1,3-Dioxolan-2-one kann beispielsweise die darin strukturell enthaltene Carbonsäurefunktion aktiviert werden , um Acylierungsreaktionen zu erleichtern. Da in diesem Fall gegebenenfalls flüchtige Ketone oder Aldehyde als Reaktionsnebenprodukt austreten, wird das Reaktionsgleichgewicht günstig in Richtung der Acylierung z.B. eines Alkohols beeinflusst (siehe "A new Synthesis of alpha-Hydroxycarboxylates and 2-Hydroxybenzoates", Khalaj, Ali; Aboofazaeli, Rem; Iranian Journal of Chemistry & Chemical Engineering (1997), 16(1), 1-3). 1,3-Dioxolan-2-one kombinieren über den Ringschluß einer alpha-Hydroxycarbonsäure den Schutz ihrer Einzelfunktionalitäten und konvertieren sie in jeweils eine Ether- und eine Lacton-Funktionalität. Hierdurch wird mitunter die Stabilität des Gesamtmoleküls entscheidend erhöht. Je nach Substituentenmuster und beabsichtigter Reaktion kann der stabilisierende oder der aktivierende Effekt im Molekül dominieren und ausgenutzt werden.

Die Verwendung von Ketonen und Aldehyden als Schutzgruppe für alpha-Hydroxycarbonsäuren ist möglich, da erstere aus den 1,3-Dioxolan-2-onen unter definierten, milden Bedingungen (sauer oder basisch) wieder abgespalten werden können. 1,3-Dioxolan-2-one sind ferner bekannte Ausgangsmaterialien zur Herstellung von alpha-Hydroxycarbonsäureestem und alpha-Hydroxycarbonsäureamiden ("Synthesis of alpha-Hydroxycarboxamides from Acetonids of alpha-Hydroxycarboxylicacids and Primary Amines" Khalaj, A.; Nahid, E. , Synthesis (1985), (12), 1153-1155).
Syntheseverfahren zur Herstellung von Dioxolanen sind beispielsweise beschrieben in "Synthesis and Configuration of Aryl-Substituted 1,3-Dioxolan-4-ones", Samoiloski, N.A.; Lapkin, LI.; Proshutinski, V.I.; Krutko, N.E.; Zhurnal Organicheskoi Khimii (1973), 9(6), 1145-1148.
Auch die Spaltung von 1,3-Dioxolan-2-onen zum 2-Hydroxycarbonsäureester wird in der Literatur bereits beschrieben (siehe "A new Synthesis of alpha-Hydroxycarboxylates and 2-Hydroxybenzoates", Khalaj, Ali; Aboofazaeli, Reza; Iranian Journal of Chemistry & Chemical Engineering (1997), 16(1), 1-3). Die Spaltung kann säure- oder basenkatalysiert ablaufen.
Weiterhin sind zahlreiche 2-Hydroxycarbonsäureester für ihre pharmakologische Wirksamkeit bekannt, so dass stets nach Wegen gesucht wird, für diese bessere und einfachere Syntheseverfahren zu entwickeln. Es ist auch bekannt, dass alpha-Hydroxycarbonsäureester selbst als Vorstufen zur Herstellung weiterer pharmakologisch wirksamer Verbindungen dienen können. Ein Beispiel hierfür sind die pharmakologisch wirksamen 2,2-Diarylglycolsäureester von Aminoalkoholen, wie z.B. das Tiotropiumsalz mit der chemischen Formel: 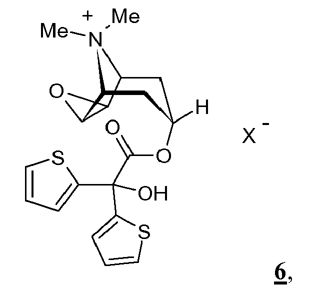 wobei X⁻ ein Anion, vorzugsweise Bromid darstellt. Tiotropiumsalze werden den Anticholinergika zugeordnet. Im Stand der Technik wird insbesondere das Tiotropiumbromid als hoch potentes Anticholinergikum beschrieben. Tiotropiumbromid ist beispielsweise aus der EP 418 716 A1 bekannt.

Alle bisher publizierten Herstellungsverfahren für Acetonide/Dioxolanone verwenden als Katalysatoren starke Säuren. Problematisch bei den bekannten Verfahren ist somit, dass säureempfindliche Verbindungen mit den bekannten klassischen sauer katalysierten Syntheseverfahren nicht zugänglich sind. Das beschränkt die Anwendbarkeit der Reaktion auf säurestabile Rohstoffe und Zielmoleküle. Im Falle der Umsetzung mit thienylsubstituierten Glycolsäure beispielsweise sind klassische Herstellverfahren durch die Instabilität dieser Komponente nicht möglich. Hier erhält man eine große Zahl an Nebenreaktionen, zum Teil auch unter Bildung von farbigen Komponenten, welche das Endprodukt verunreinigen und einen hohen Aufwand für die Reinigung nach sich ziehen.

Der vorliegenden Erfindung liegt somit die Aufgabe zugrunde, ein verbessertes, mildes, technisch anwendbares Syntheseverfahren bereitzustellen, welches einen synthetischen Zugang auch zu 1,3-Dioxolanen bzw. 2-Hydroxycarbonsäureestern erlaubt, die temperatur-und/oder säureempfindliche Funktionalitäten aufweisen.

### Beschreibung der Erfindung

Die vorliegende Erfindung betrifft in einem ersten Aspekt ein Verfahren (Reaktion 1a) zur Herstellung von 1,3-Dioxolan-2-on der Formel 3 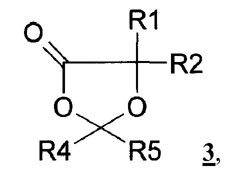 worin
- R₁ und R₂: unabhängig voneinander jeweils Wasserstoff oder einen Cycloalkyl-, Aryl-oder Heteroaryl-Rest bedeuten, wobei der Cycloalkyl-, Aryl- oder Heteroaryl-Rest jeweils gegebenenfalls ein- oder mehrfach substituiert sein kann;

- R₄ und R₅: jeweils unabhängig voneinander ausgewählt sind aus Wasserstoff, C₁-C₆-Alkyl, oder R₄ und R₅ zusammen einen gesättigten oder ein- oder mehrfach ungesättigten carbocyclischen oder heterocyclischen Ring, der ein oder mehrere Heteroatom, ausgewählt aus S, N oder O, enthalten kann, der gegebenenfalls jeweils unabhängig voneinander ein oder mehrfach substituiert sein kann, bilden, wobei R₄ und R₅ nicht gleichzeitig Wasserstoff sein können;
wobei ein 2-Hydroxycarbonsäureester der Formel 1 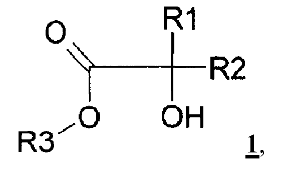 worin
- R₁ und R₂: wie oben definiert sind und
- R₃: Methyl, Ethyl, Propyl, Isopropyl, Isopropenyl, Butyl, N-Succinimid, N-Phtalimid, Phenyl, Nitrophenyl, Fluorophenyl, Pentafluorophenyl, Vinyl, 2-Allyl bedeutet,
in einem geeigneten Lösemittel unter Zusatz eines geeigneten basischen Katalysators, vorzugsweise eines Katalysators ausgewählt aus der Gruppe der tert.-Alkoholate, in Gegenwart von Zeolith in einem Schritt mit einer Verbindung der Formel **2** 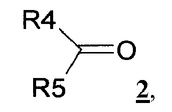 umgesetzt wird, worin R₄ und R₅ wie oben definiert sind.

Die Erfindung betrifft weiterhin in einem zweiten Aspekt ein Verfahren (Reaktion 1a + Reaktion 1b) zur Herstellung einer Verbindung der Formel **5** 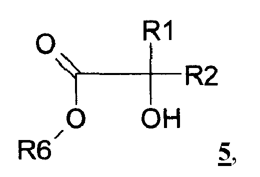 worin
- R₁ und R₂: wie oben definiert sind;
- R₆: einen organischen Rest bedeutet,
wobei ein 2-Hydroxycarbonsäureester der Formel 1 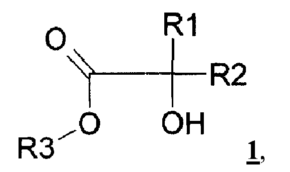 worin
- R₁ und R₂: wie oben definiert sind;
- R₃: unterschiedlich zu R₆ ist und ausgewählt ist aus Methyl, Ethyl, Propyl, Isopropyl, Isopropenyl, Butyl, N-Succinimid, N-Phtalimid, Phenyl, Nitrophenyl, Fluorophenyl, Pentafluorophenyl, Vinyl, 2-Allyl,
in einem ersten Schritt in einem geeigneten Lösemittel unter Zusatz eines geeigneten basischen Katalysators, vorzugsweise eines Katalysators ausgewählt aus der Gruppe der tert.-Alkoholate, in Gegenwart von Zeolith in einem Schritt mit einer Verbindung der Formel **2** 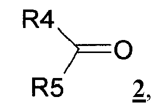 worin R₄ und R₅ wie oben definiert sind,
zu einer Verbindung der Formel **3** 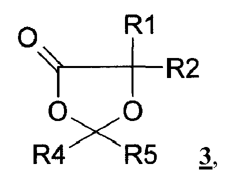 worin R₁, R₂, R₄ und R₅ die vorstehend genannten Bedeutungen haben,
umgesetzt wird, und worin
die Verbindung der Formel **3** in einem zweiten Schritt (Reaktion 1b) in einem geeigneten Lösemittel unter Zusatz eines geeigneten basischen Katalysators, vorzugsweise einem Katalysator ausgewählt aus der Gruppe der tert.-Alkoholate, in Gegenwart von Zeolith mit einer Verbindung der Formel **4**

**R₆-O-H** , 4

worin
- R₆: einen organischen Rest bedeutet, der von R₃ unterschiedlich ist,
zu einer Verbindung der Formel **5** umgesetzt wird.

Die Erfindung betrifft weiterhin in einem dritten Aspekt ein Verfahren (Reaktion 2) zur Herstellung einer Verbindung der Formel **5** 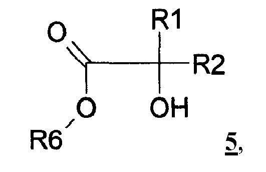 worin
R₁ und R₂ wie oben definiert sind und
R₆ wie oben definiert ist;
wobei ein 2-Hydroxycarbonsäureester der Formel **1** 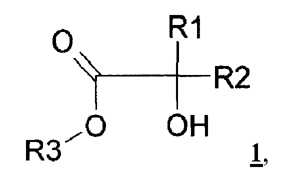 worin
R₁ und R₂ und R₃ wie oben definiert sind;
in einem Schritt (Reaktion 2) mit einer Verbindung der Formel **2**, insbesondere Aceton, das als Lösemittel dient, und einer Verbindung der Formel **4**

**R₆-O-H** , 4

worin
R₆ einen organischen Rest bedeutet, der von R₃ unterschiedlich ist,
unter Zusatz eines geeigneten basischen Katalysators, vorzugsweise einem Katalysator ausgewählt aus der Gruppe der tert.-Alkoholate, in Gegenwart von Zeolith zu einer Verbindung der Formel **5** umgesetzt wird.

### Detaillierte Beschreibung der Erfindung

### Begriffsdefinitionen

Der Begriff "Aryl" bzw. "Aryl-Rest" bedeutet einen 6- bis 10-gliedrigen aromatischen Carbocyclus und umfasst beispielsweise Phenyl und Naphthyl. Andere Begriffe, welche als Bestandteil den Begriff "Aryl" enthalten, weisen dieselbe Bedeutung für den Aryl-Bestandteil auf. Beispiele für diese Komponenten sind: Arylalkyl, Aryloxy oder Arylthio.

Der Begriff "Heteroaryl" bzw. "Heteroaryl-Rest" bezeichnet einen stabilen 5- bis 8-gliedrigen, bevorzugt 5- oder 6-gliedrigen monocyclischen oder 8- bis 11-gliedrigen bicyclischen aromatischen Heterocyclus. Jeder Heterocyclus besteht aus Kohlenstoffatomen und 1 bis 4 Heteroatomen, ausgewählt aus Stickstoff, Sauerstoff und Schwefel. Beispiele von Heteroarylen sind: Pyridyl, Pyrimidinyl, Pyrazinyl, Pyridazinyl, Pyrrolyl, Imidazolyl, Pyrazolyl, Thienyl, Furanyl, Isoxazolyl, Isothiazolyl, Oxazolyl, Thiazolyl, Oxadiazolyl, Thiadiazolyl, Chinolinyl, Isochinolinyl, Indolyl, Benzimidazolyl, Benzofuranyl, Benzoxazolyl, Benzisoxazolyl, Benzpyrazolyl, Benzothiofuranyl, Benzothiazolyl, Chinazolinyl, Indazolyl, oder kondensiertes Heteroaryl wie Cyclopentenopyridin, Cyclohexanopyridin, Cyclopentanopyrimidin, Cyclohexanopyrimidin, Cyclopentanopyrazin, Cyclohexanopyrazin, Cyclopentanopyridazin oder auch Cyclohexanopyridazin.

Als "Alkyl" bzw. "Alkylgruppen" sowie Alkylgruppen, welche Bestandteil anderer Reste sind, werden verzweigte und unverzweigte Alkylgruppen mit 1 bis 6 Kohlenstoffatomen bezeichnet. Beispielsweise werden genannt: Methyl, Ethyl, Propyl, Butyl, Pentyl, Hexyl. Sofern nicht anders angegeben, sind von den vorstehend genannten Bezeichnungen Propyl, Butyl, Pentyl und Hexyl sämtliche der möglichen isomeren Formen umfaßt. Beispielsweise umfaßt die Bezeichnung Propyl die beiden isomeren Reste n-Propyl und iso-Propyl, die Bezeichnung Butyl die isomeren Reste n-Butyl, iso-Butyl, sek. Butyl und tert.-Butyl.

Als "Alkoxy" oder "Alkyloxygruppen", werden verzweigte und unverzweigte Alkylgruppen mit 1 bis 6 Kohlenstoffatomen bezeichnet, die über ein Sauerstoffatom verknüpft sind. Beispielsweise werden genannt: Methoxy, Ethoxy, Propoxy, Butoxy, Pentoxy, Hexoxy. Sofern nicht anders angegeben, sind von den vorstehend genannten Bezeichnungen sämtliche der möglichen isomeren Formen umfaßt.

Bezeichnungen, wie "Fluorphenyl" und "Nitrophenyl", stehen für Phenylringe, die durch Fluor oder NO₂ substituiert sind. Hierbei sind alle möglichen Isomeren (ortho, meta oder para) umfasst, wobei der para- und der meta-Substitution besondere Bedeutung zukommt.

Als "carbocyclischer Ring" oder "Cycloalkylgruppen" werden Cycloalkylreste mit 3 bis 6 Kohlenstoffatomen, beispielsweise Cyclopropyl, Cyclobutyl, Cyclopentyl oder Cyclohexyl bezeichnet.

Unter dem Begriff "Heterocyclus" wird ein stabiler 5- bis 8-gliedriger, aber bevorzugt 5-oder 6-gliedriger monocyclischer oder 8- bis 11-gliedriger bicyclischer Heterocyclus verstanden, der entweder gesättigt oder ungesättigt ist und auch aromatisch sein kann, sofern dies chemisch im jeweiligen Zusammenhang möglich ist. Jeder Heterocyclus besteht aus Kohlenstoffatomen und 1 bis 4 Heteroatomen, ausgewählt aus Stickstoff, Sauerstoff und Schwefel. Beispiele von Hetercyclen sind Pyrrolinyl, Pyrrolidinyl, Pyrazolinyl, Pyrazolidinyl, 1,2,5,6-Tetrahydropyridinyl, Piperidinyl, Morpholinyl, Thiomorpholinyl, Pyranyl, Thiopyranyl, Piperazinyl, Indolinyl oder auch 1,2,3,3a,4,6a-Hexahydrocyclopenta[c]pyrrolyl .

Der Begriff "Stickstoff" und "Schwefel" sowie die jeweiligen Elementsymbole, umfassen jegliche oxidierte Form dieser und auch quaternäre Formen eines basischen Stickstoffatoms sollen umfasst sein.

### Bevorzugte Ausführungformen

Nachfolgend werden die erfindungsgemäßen Verfahrensvarianten im Einzelnen beschrieben. Diese sind im nachfolgenden Reaktionsschema 1 schematisiert:

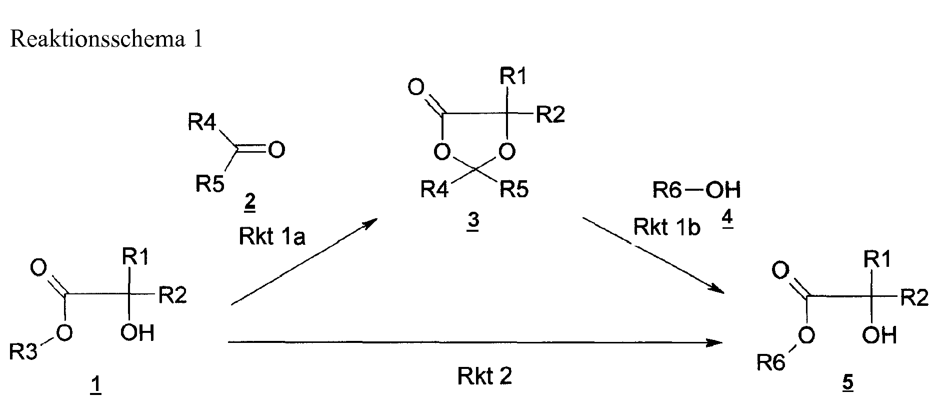

Die Reste R₁ bis R₆ haben die vorstehend angegebenen Bedeutungen.

**Reaktion 1a:** Das erfindungsgemäße Verfahren (Reaktion 1a) gemäß dem ersten Aspekt der Erfindung betrifft die Umsetzung eines 2-Hydroxycarbonsäureesters der allgemeinen Formel **1** mit einer Verbindung der Formel **2** zu einem 1,3-Dioxolan-2-on der allgemeinen Formel **3.** Dies ist im nachfolgenden Reaktionsschema 2 dargestellt:

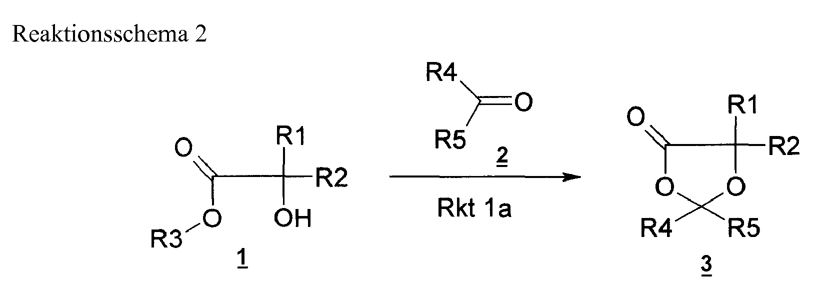

Die Reste R₁ bis R₅ haben die vorstehend angegebenen Bedeutungen.

Nach einer besonders bevorzugten Ausführungsform der Erfindung ist R₃ bevorzugt ausgewählt aus Methyl, Ethyl, Propyl und Butyl, besonders bevorzugt Methyl.

Besonders bevorzugt wird R₁ und R₂ jeweils ausgewählt aus einem Heteroaryl-Rest, der jeweils gegebenenfalls ein- oder mehrfach substituiert sein kann. Bevorzugte Substituenten sind Halogenatome, wie Fluor, Chlor, Brom oder Jod, -CN, C₁-C₄-Alkyl, C₁-C₄-Alkoxy, Hydroxy oder CF₃. Ganz besonders bevorzugt stellen R₁ und R₂ beide denselben Heteroaryl-Rest dar, insbesondere sind R₁ und R₂ beide Thienyl-Reste.

Bevorzugt sind R₄ und R₅ unabhängig ausgewählt aus C₁-C₆-Alkyl, besonders bevorzugt Methyl, Ethyl, Butyl oder Pentyl. Nach einer bevorzugten Ausführungsform stellen R₄ und R₅ beide die gleiche Gruppe dar, jedoch nicht gleichzeitig Wasserstoff, besonders bevorzugt sind R₄ und R₅ beide Methyl.

Nach einer weiteren bevorzugten Ausführungsform bilden R₄ und R₅ zusammen einen gesättigten oder ungesättigten carbocyclischen oder heterocyclischen Ring. Der carbocyclische Ring ist bevorzugt ausgewählt aus Cyclohexyl und Cyclopentyl. Der heterocyclische Rest ist bevorzugt ausgewählt aus Piperidinyl-, Chinuclidinyl, Tropinyl und Pyrrolidinyl, jeweils gegebenenfalls substituiert mit ein oder mehr Substituenten. Diese sind vorzugsweise ausgewählt aus Halogenatomen, wie Fluor, Chlor, Brom oder Jod, -CN, C₁-C₄-Alkyl, C₁-C₄-Alkoxy, Hydroxy, CF₃ und -O-COR', wobei R' einen Rest darstellt, ausgewählt aus der Gruppe, bestehend aus C₁-C₄-Alkyl, Benzyl und Phenylethyl, der jeweils substituiert sein kann durch Hydroxy, Hydroxymethyl oder Methoxy, besonders bevorzugt bilden R₄ und R₅ zusammen einen Pyridinyl-Ring.

Wenn R₄ und R₅ ein Ringsystem ausbilden, kann bei der Verbindung der Formel **2** auch ein aromatischer carbocyclischer oder heterocyclischer Ring ausgebildet werden. Beispiele hierfür sind Benzyl, Pyrrol, Furan, Thiophen. Nach der Umsetzung der Verbindung der Formel **2** zum 1,3-Dioxolan-2-on verliert das Ringsystem seinen aromatischen Charakter aufgrund des Brücken-Kohlenstoffatoms zwischen den beiden Ring-Sauerstoffen des Dioxolans.

Nach einer besonders bevorzugten Ausführungsform der Erfindung bilden R₄ und R₅ gemeinsam mit einem Stickstoffatom (auch als quateräres Ammoniumsalz möglich ) einen heterocyclischen Ring ausgewählt aus Pyrrol, Pyrrolin, Pyrrolidin, Pyridin, Piperidin, Morpholin, 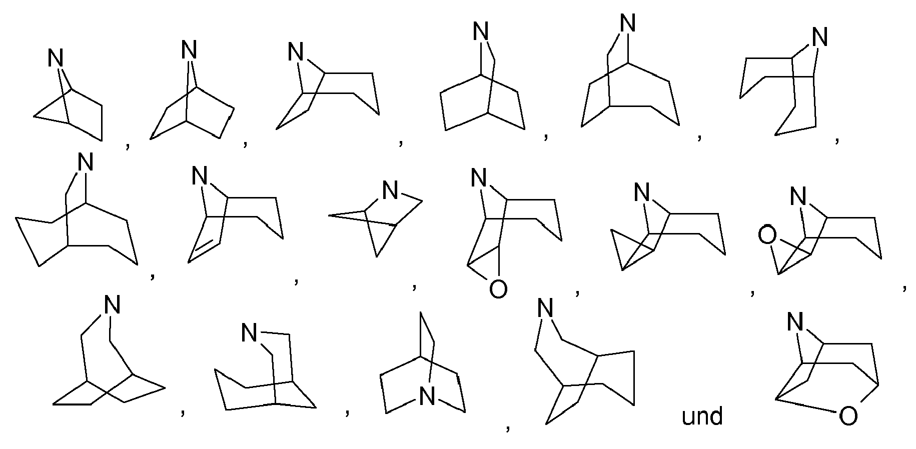 der gegebenenfalls substituiert sein kann durch einen oder mehrere Reste, bevorzugt einen Rest ausgewählt aus der Gruppe bestehend aus OH, F, Methyl, Ethyl, Methoxy und -O-COR', wobei R' einen Rest darstellt, ausgewählt aus der Gruppe, bestehend aus C₁-C₄-Alkyl, Benzyl und Phenylethyl, der jeweils substituiert sein kann durch Hydroxy, Hydroxymethyl oder Methoxy.

Die Verbindungen der Formel **2** sind nicht besonders beschränkt; es können bekannte Ketone oder Aldehyde eingesetzt werden, sofern diese die Bildung eines 1,3-Dioxolan-2-ons nicht beeinträchtigen. Sterisch besonders anspruchsvolle Reste sind demnach nicht bevorzugt. Beispielhafte Ketone sind Cyclohexanon, Aceton. Beispielhafte Aldehyde sind Isobutyraldehyd, Benzaldehyd, N-Methy-1,4-piperidon, Tropanon, Piperidon, Chinuclidinon.

Erfindungsgemäß ist eine besonders schonende Umsetzung möglich, da der Katalysator, bevorzugt ein Katalysator ausgewählt aus der Gruppe der tert.-Alkoholate, zusammen mit einem Zeolith eingesetzt wird. Unter tert.-Alkoholaten werden Alkoholate verstanden, die sterisch anspruchsvolle Alkylgruppen aufweisen, in denen wenigstens ein quartäres Kohlenstoffzentrum enthalten ist. Unter quartärem Kohlenstoffzentrum werden dabei Kohlenstoffzentren verstanden, die kein Wasserstoffatom tragen, sondern mit 3 bis 4 Alkylgruppen substituiert sind. Kohlenstoffzentren, die mit 3 Alkylgruppen substituiert sind, tragen als vierten Substituenten vorzugsweise die Alkoholatgruppe. Geeignete tert.-Alkoholate sind bevorzugt Alkali- oder Erdalkalialkoholate, besomders bevorzugt Natrium- oder Kalium-tert.-butylat oder Natrium- oder Kalium-tert.-Amylat.

Besonders bevorzugte Zeolithe sind Molekularsiebe, die ausgewählt sind aus der Gruppe der Molekularsiebe mit basischem Charakter, bestehend aus Alkali- oder Erdalkali-haltigen Alumosilikaten, wie Natrium- oder Kalium-haltigen Alumosilikaten, bevorzugt Molekularsiebe mit der Summenformel Na₁₂[(AlO₂)₁₂(SiO₂)₁₂] x H₂O oder oder Na₁₂Al₁₂Si₁₂O₃₆ x n H₂O, wobei n bevorzugt < 6 , Molekularsiebs Typ 4A erfindungsgemäß besonders bevorzugt ist.

Die Umsetzung der Verbindung der Formel **1** mit der Verbindung der Formel **2** zur Verbindung der Formel **3** erfolgt bevorzugt in Lösung. Die erhaltene Lösung wird in der Regel bis zur vollständigen Umsetzung gerührt. Hierbei kann bei Raumtemperatur (ca. 23°C) oder gegebenenfalls auch bei leicht erhöhter Temperatur in einem Bereich von 25-50°C gearbeitet werden. Dies hängt von den Reaktanten und vom gewählten Katalysator-Typ ab. Die Umsetzung läuft unter basischen Reaktionsbedingungen ab und funktioniert auch bei Raumtemperatur und darunter. Bevorzugt erfolgt die Umsetzung unter milden Bedingungen bei einer Temperatur von etwa 30°C, besonders bevorzugt in einem Bereich von etwa 0 bis etwa 30°C.

Als Lösemittel kommen vorzugsweise aprotische organische Lösemittel, bevorzugt schwach polare organische Lösemittel in Frage. Als Lösemittel kommen erfindungsgemäß besonderes bevorzugt Aceton, Pyridin, Acetonitril und Tetrahydrofuran in Betracht, wobei Aceton, Acetonitril und Tetrahydrofuran bevorzugt verwendet werden. Von besonderem Vorteil kann es sein, wenn die Verbindung der Formel **2** gleichzeitig als Reaktant und Lösemittel fungieren kann. Dies ist beispielsweise bei der Verwendung von Aceton als Verbindung der Formel **2** der Fall und ist besonders vorteilhaft, wenn der alpha-Hydroxycarbonsäureester der Formel **1** in diesem Lösemittel löslich ist.

Die erhaltene Lösung wird in der Regel bis zur vollständigen Umsetzung gerührt. Nach Beendigung der Umsetzung werden die Verbindungen der Formel **3** aus der Lösung isoliert. Die erhaltenen Produkte können, falls erforderlich, durch Umkristallisation aus einem geeigneten Lösemittel gereinigt werden. Das erhaltene Produkt wird isoliert und gegebenenfalls im Vakuum getrocknet.

Das erfindungsgemäße Verfahren (Reaktion 1a) verläuft selektiv, Nebenreaktionen werden weitgehend unterbunden.

Nach einer besonders bevorzugten erfindungsgemäßen Ausführungsform werden durch das erfindungsgemäße Verfahren 1,3-Dioxolan-2-one mit einer Grundstruktur vom Typ A und 1,3-Dioxolan-2-one mit einer Grundstruktur vom Typ B hergestellt:

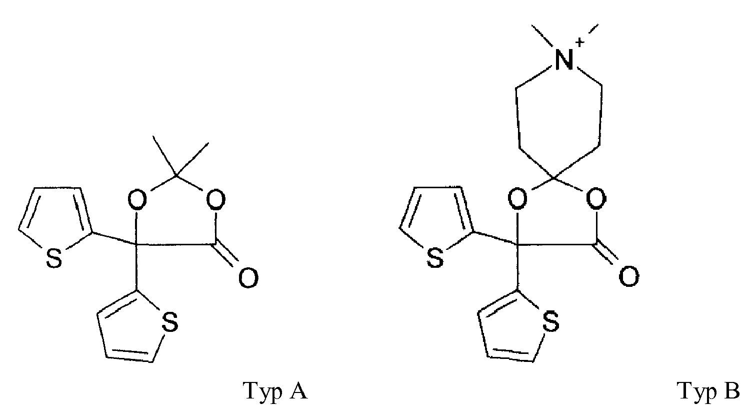

Die Herstellung dieser Grundstrukturen vom Typ A und Typ B war bislang auf klassischem säurekatalysiertem Wege nicht möglich (siehe "Synthesis and Selective Activity of Cholinergic Agents with Rigid Skeletons", Shoji Takemura, Yasuyoshi Miki, Mariko Hoshida, Mayumi Shibano, Aritomo Suzuki; Chem. Pharm. Bull. 29(10) 3019-3025 (1981)), da sie und ihre Dithienylglycolsäure-Vorläufer säurempfindlich sind. Demnach können derartige Verbindung durch das erfindungsgemäße Verfahren nunmehr zur Verfügung gestellt werden.

Die vorliegende Erfindung betrifft demnach auch ein Verfahren zur Herstellung säureempfindlicher 2-Hydroxycarbonsäureester **5**, wobei auch als Ausgangsmaterial ein säureempfindlicher 2-Hydroxycarbonsäureester **1** vorliegen kann.

1,3-Dioxolan-2-one des Typs B wirken überraschenderweise als Anticholinergika am muscarinischen Rezeptor, ähnlich wie Tiotropium-Salze, und können bei bestimmten Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Pharmakologisch aktive Benzilsäure und Mandelsäure-Analoga wurden beispielsweise in "Synthesis and Selective Activity of Cholinergic Agents with Rigid Skeletons", Shoji Takemura, Yasuyoshi Miki, Mariko Hoshida, Mayumi Shibano, Aritomo Suzuki; Chem. Pharm. Bull. 29(10) 3019-3025 (1998)) beschrieben.

Insbesondere stellen die Verbindungen mit der Formel **610**: 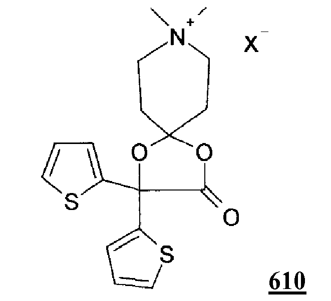 hoch potente anticholinerge Wirkstoffe dar, wobei X⁻ ein einfach geladenes Anion darstellt. Beispielsweise kann es sich bei diesem Anion um Chlorid, Bromid, Jodid, Sulfat, Phosphat, Methansulfonat, Nitrat, Maleat, Acetat, Citrat, Fumarat, Tartrat, Oxalat, Succinat, Benzoat oder p-Toluolsulfonat handeln.

Dementsprechend betrifft ein weiterer Aspekt der vorliegenden Erfindung die Verbindungen der Formel **610** , wobei X⁻ Chlorid, Bromid, Jodid, Sulfat, Phosphat, Methansulfonat, Nitrat, Maleat, Acetat, Citrat, Fumarat, Tartrat, Oxalat, Succinat, Benzoat oder p-Toluolsulfonat darstellt, wobei die Substituenten am Stickstoff ausgewählt sind aus Methyl, Ethyl, n-Alkyl, iso-Alkyl, Alkoxyalkyl, Hydroxyalkyl und die Thienyl-Reste gegebenenfalls ein oder mehrere Substituenten aufweisen können, die ausgewählt sind aus der Gruppe bestehend aus Fluor, Chlor, Brom, Jod, -CN, C₁-C₄-Alkyl, C₁-C₄-Alkoxy, Hydroxy und CF₃.

Weitere Anwendungen von erfindungsgemäß herstellbaren 1,3-Dioxolan-2-on-Verbindungen liegen beispielsweise im synthetischen Bereich zur Herstellung von pharmakologisch aktiven Carbonsäureestern, wie z.B. Tiotropiumbromid. Dies kann beispielsweise aus/über Verbindungen vom oben beschriebenen Typ A erfolgen.

1,3-Dioxolan-2-one können insbesondere dann Verwendung finden, wenn säureempfindliche Verbindungen vorliegen, wie säureempfindliche Carbonsäureester, z.B. 2,2-Dithienylglycolsäuremethylester. Zudem können die 1,3-Dioxolan-2-one wieder in andere (z.B. pharmakologisch aktive) Carbonsäureester oder Carbonsäuresalze umgewandelt werden.

Das erfindungsgemäße Verfahren (Reaktion 1a) erlaubt eine Umsetzung unter sehr schonenden Bedingungen, die im Vergleich zu Reaktionen in hochpolaren aprotischen Lösemitteln weniger Nebenreaktionen und damit zusammenhängend eine höhere Ausbeute zur Folge haben. Dies kann beispielsweise zur Synthese empfindlicher säurelabiler und/oder temperaturempfindlicher Systeme herangezogen werden.

### Reaktion 1b

Das erfindungsgemäße Verfahren (Reaktion 1b) bezieht sich weiterhin auf die Herstellung von 2- oder alpha-Hydroxycarbonsäureestern der allgemeinen Formel **5** ausgehend von einem 1,3-Dioxolan-2-on der allgemeinen Formel **3** unter basischen Reaktionsbedingungen. Derartige Umsetzungen sind aus dem Stand der Technik bekannt. Dies wird im nachfolgenden Reaktionsschema 3 dargestellt:

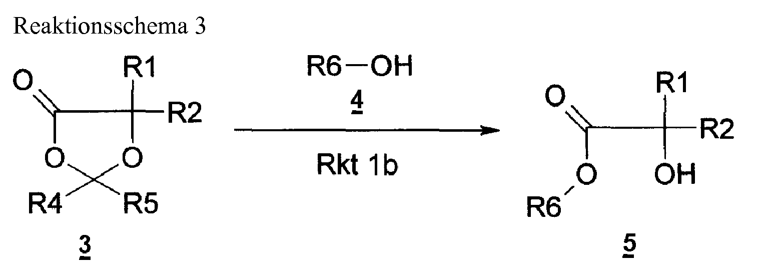

Die Reste R₁ bis R₆ haben die vorstehend angegebenen Bedeutungen.

Gemäß der Erfindung wird vorzugsweise die Verbindung der Formel **3** in einem Schritt (Reaktion 1b) in einem geeigneten Lösemittel unter Zusatz eines geeigneten basischen Katalysators, bevorzugt ein Katalysator ausgewählt aus der Gruppe der tert.-Alkoholate, in Gegenwart von Zeolith mit einer Verbindung der Formel **4**

**R₆-O-H,** 4

worin
- R₆: einen organischen Rest bedeutet, der von R₃ unterschiedlich ist,
zu einer Verbindung der Formel **5** 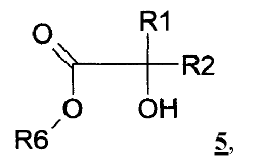 worin R₁, R₂ und R₆ die vorstehend genannten Bedeutungen haben,
umgesetzt.

Die Reaktionstemperatur hängt von den Reaktanden und vom gewählten Katalysator-Typ ab. Die Umsetzung läuft unter basischen Reaktionsbedingungen ab und funktioniert auch bei Raumtemperatur und darunter. Bevorzugt erfolgt die Umsetzung unter milden Bedingungen bei einer Temperatur von etwa 30°C, besonders bevorzugt in einem Bereich von etwa 0 bis etwa 30°C. Als Lösemittel kommen vorzugsweise aprotische organische Lösemittel, bevorzugt schwach polare organische Lösemittel in Frage. Als Lösemittel kommen erfindungsgemäß besonderes bevorzugt Aceton, Pyridin, Acetonitril und Tetrahydrofuran in Betracht, wobei Aceton, Acetonitril und Tetrahydrofuran besonders bevorzugt verwendet werden.

Für weitere Einzelheiten wird beispielsweise auf "A new Synthesis of alpha-Hydroxycarboxylates and 2-Hydroxybenzoates", Khalaj, Ali; Aboofazaeli, Reza; Iranian Journal of Chemistry & Chemical Engineering (1997), 16(1), 1-3 verwiesen.

### Reaktion 2

Das erfindungsgemäße Verfahren (Reaktion 2) betrifft ferner die Herstellung von 2-Hydroxycarbonsäureestem der allgemeinen Formel **5** über eine Zwischenstufe in Form eines Derivats des 1,3-Dioxolan-2-ons der allgemeinen Formel **3** unter basischen Reaktionsbedingungen durch Umesterung des entsprechenden Esters der allgemeinen Formel **1**. Das 1,3-Dioxolan-2-on der Formel **3** wird in diesem Fall nicht als Zwischenprodukt isoliert sondern direkt zum neuen Ester weiterverarbeitet. Dies ist im nachfolgenden Reaktionsschema 4 dargestellt: 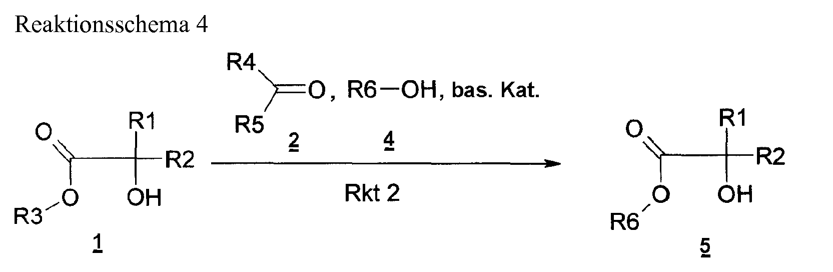 Die Reste R₁ bis R₆ haben die vorstehend angegebenen Bedeutungen.

Hierbei findet eine Umesterung oder Transacylierung des Esters unter basischen Reaktionsbedingungen statt.

Wenn das Zwischenprodukt in Form des 1,3-Dioxolan-2-ons der Formel **3** isoliert werden soll, wird wie oben beschrieben bei der Reaktion 1a vorgegangen (Reaktion 1a -> Zwischenprodukt wird isoliert -> Reaktion 1b), an welche sich die Reaktion 1b anschließen kann.

Soll das Zwischenprodukt in Form des 1,3-Dioxolan-2-ons der Formel **3** jedoch nicht isoliert werden, wird das erfindungsgemäße Verfahren (Reaktion 2) vorzugsweise in einem Schritt durchgeführt (Reaktion 1a + Reaktion 1b = Reaktion 2). Hierzu wird die Verbindung der Formel **1** unter Verwendung der Verbindung der Formel **2** und der Verbindung der Formel **4** quasi in einem Schritt ("Eintopfverfahren") zu einer Verbindung der Formel **5** umgesetzt.

Selbstverständlich können die Reaktion 1a und die Reaktion 1b, wie oben beschrieben, auch nacheinander durchgeführt werden, ohne das Zwischenprodukt zu isolieren; dies ist jedoch weniger bevorzugt.

Nach einer besonders bevorzugten Ausführungsform der Erfindung ist R₃ bevorzugt ausgewählt aus Methyl, Ethyl, Propyl und Butyl, besonders bevorzugt Methyl oder Ethyl.

Besonders bevorzugt werden R₁ und R₂ jeweils ausgewählt aus einem Heteroaryl-Rest, der jeweils gegebenenfalls ein- oder mehrfach substituiert sein kann. Bevorzugte Substituenten sind Halogenatome, wie Fluor, Chlor, Brom oder Jod, -CN, C₁-C₄-Alkyl, C₁-C₄-Alkoxy, Hydroxy oder CF₃. Ganz besonders bevorzugt stellen R₁ und R₂ beide denselben Heteroaryl-Rest dar, insbesondere sind R₁ und R₂ beide Thienyl-Reste.

Nach einer besonders bevorzugten Ausführungsform der Erfindung bilden R₄ und R₅ gemeinsam mit einem Stickstoffatom einen heterocyclischen Ring ausgewählt aus Pyrrol, Pyrrolin, Pyrrolidin, Pyridin, Piperidin, Morpholin, Chinuclidin 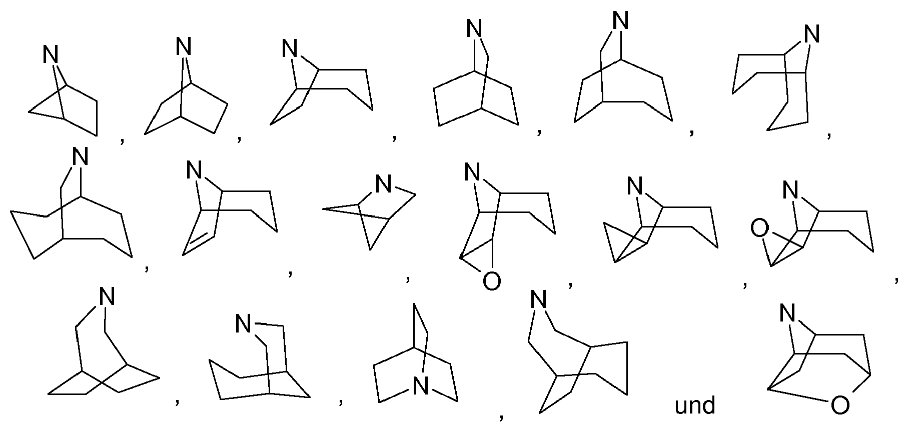 der gegebenenfalls substituiert sein kann durch einen oder mehrere Reste, bevorzugt einen Rest, ausgewählt aus der Gruppe, bestehend aus OH, F, Methyl, Ethyl, Methoxy und -O-COR', wobei R' einen Rest darstellt, ausgewählt aus der Gruppe, bestehend aus C₁-C₄-Alkyl, Benzyl und Phenylethyl, der jeweils substituiert sein kann durch Hydroxy, Hydroxymethyl oder Methoxy.

Die Verbindung der Formel **3** wird in dieser bevorzugten erfindungsgemäßen Verfahrensvariante (Reaktion 2) ausgewählt aus Aceton. Hierbei handelt es sich um ein aprotisches Lösemittel, welches gleichzeitig als Lösemittel und Verbindung **3** fungiert. Jedoch kann zusätzlich ein weiteres aprotisches Lösemittel eingesetzt werden, wie beispielsweise Acetonitril. Dies ist jedoch nicht bevorzugt.

Erfindungsgemäß ist eine besonders schonende Umsetzung möglich, da der Katalysator, bevorzugt ein Katalysator ausgewählt aus der Gruppe der tert.-Alkoholate, zusammen mit einem Zeolith eingesetzt wird. Unter tert.-Alkoholaten werden Alkoholate verstanden, die sterisch anspruchsvolle Alkylgruppen aufweisen, in denen wenigstens ein quartäres Kohlenstoffzentrum enthalten ist. Unter quartärem Kohlenstoffzentrum werden dabei Kohlenstoffzentren verstanden, die kein Wasserstoffatom tragen, sondern mit 3 bis 4 Alkylgruppen substituiert sind. Kohlenstoffzentren, die mit 3 Alkylgruppen substituiert sind, tragen als vierten Substituenten vorzugsweise die Alkoholatgruppe. Geeignete tert.-Alkoholate sind bevorzugt Alkali- oder Erdalkalialkoholate, besomders bevorzugt Natrium- oder Kalium-tert.-butylat oder Natrium- oder Kalium-tert.-Amylat.

Besonders bevorzugte Zeolithe sind Molekularsiebe, die ausgewählt sind aus der Gruppe der Molekularsiebe mit basischem Charakter, bestehend aus Alkali- oder Erdalkali-haltigen Alumisilikaten, wie Natrium- oder Kalium-haltigen Alumosilikaten, bevorzugt Molekularsiebe mit der Summenformel Na₁₂[(AlO₂)₁₂(SiO₂)₁₂] x H₂O oder Na₁₂Al₁₂Si₁₂O₃₆ x n H₂O, wobei n bevorzugt < 6 , ( Verwendung des Molekularsiebs Typ 4A ) erfindungsgemäß besonders bevorzugt ist.

Von besonderem Vorteil hat es sich herausgestellt, wenn als Reaktionsmedium eine Kombination Aceton/tert.-Alkoholat bzw. Acetonitril/Aceton/tert-Alkoholat für die erfindungsgemäße Umesterungsreaktion eingesetzt wird. Durch die Gegenwart von Zeolith werden außerdem die üblicherweise auftretenden Selbstkondensationsreaktionen des Acetons oder des Acetonitrils unterbunden. Durch die Verwendung von Zeolith wird ferner der freiwerdende Alkohol dem Gleichgewicht effektiv entzogen.

Der Alkohol der Formel **4** ist variabel, aber in der Praxis auf primäre oder sekundäre Alkohole beschränkt. Für die chemische Umsetzung kann praktisch jeder dem Fachmann bekannte primäre oder sekundäre Alkohol zum Einsatz kommen, da auch mit jedem dieser Alkohole aufgrund der Verschiebung des Reaktionsgleichgewichts durch Entzug des gebildeten Alkohols eine Umsetzung erfolgt.
Die Reaktivität der Alkohole ist bei primären Alkoholen am größten. Tertiäre Alkohole nehmen an dieser Reaktion nicht teil.
Der Rest R₆ im Alkohol der Formel **4** stellt einen weitgehend frei wählbaren organischen Rest dar, welcher nach der Umsetzung den im erzeugten 2-Hydroxycarbonsäureester der Formel **5** umgeesterten Rest darstellt. Durch Wahl des Alkohols kann somit das gewünschte Endprodukt festgelegt werden. Der organische Rest R₆ kann beispielsweise sein: Ein verzweigtes oder unverzweigtes Alkyl, ein gesättigter oder ungesättigter Carbocyclus, Heterocyclus, Bicyclus, Tricyclus, ein kondensiertes cyclisches System und viele andere mehr sowie Kombinationen hiervon. Beispiele für Heterocyclen sind unter den Begriffsdefinitionen angegeben.

Besonders bevorzugte Heterocyclen, die für R₆ verwendet werden können, sind ausgewählt aus Pyrrol, Pyrrolin, Pyrrolidin, Pyridin, Piperidin, Morpholin, Scopin, N-Methylscopinium 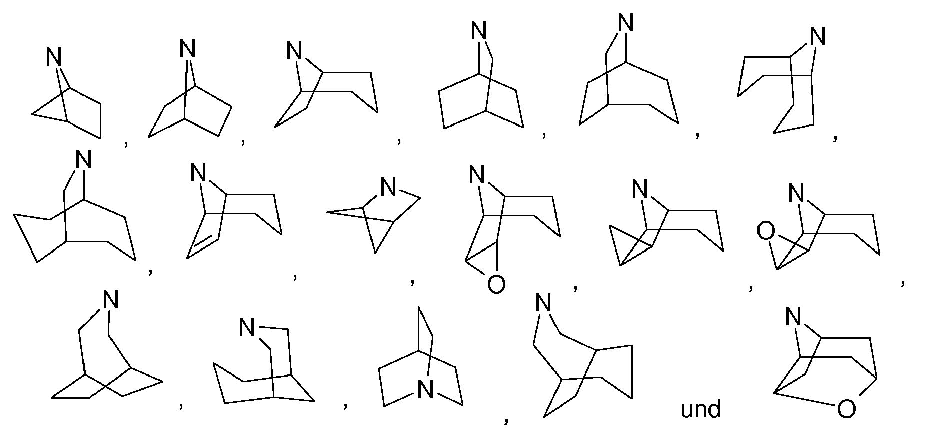 die gegebenenfalls substituiert sein können durch einen oder mehrere, bevorzugt einen Rest ausgewählt aus der Gruppe bestehend aus OH, F, Methyl, Ethyl, Methoxy und -O-COR', wobei R' einen Rest, ausgewählt aus der Gruppe, bestehend aus C₁-C₄-Alkyl, Benzyl und Phenylethyl, der jeweils substituiert sein kann durch Hydroxy, Hydroxymethyl oder Methoxy.

Der Alkohol ist demnach im Rahmen der Erfindung nicht beschränkt, sofern dieser keine funktionelle Gruppe enthält, welche in die Reaktion eingreift und sofern er kein tertiärer Alkohol ist.

Ein besonderer Vorteil des neuen Herstellverfahrens unter Verwendung der KatalysatorKombination, bestehend aus dem tert.-Alkoholat-imprägnierten Zeolith ergibt sich beispielsweise für das basenempfindliche Scopin. Die Reaktionsbedingungen sind so sanft, dass die Umlagerung zum entsprechenden Scopolin vermieden werden kann.

Statt der Verbindung der Formel **4** R₆-OH kann auch eine Verbindung der Formel **4'** R₆-NH₂ eingesetzt werden, um ein entsprechendes 2-Hydroxycarbonsäureamid herzustellen. Dies trifft für die Reaktion 1b und die Reaktion 2 der Erfindung zu, wobei dieselben Lösemittel und Bedingungen wie für den Alkohol beschrieben auch im Falle der Verwendung eines Amins zum Einsatz kommen können. Das Reaktionsschema 5 für die Reaktion 1b mit der Verbindung der Formel **4'** R₆-NH₂ ergibt sich dann wie folgt:

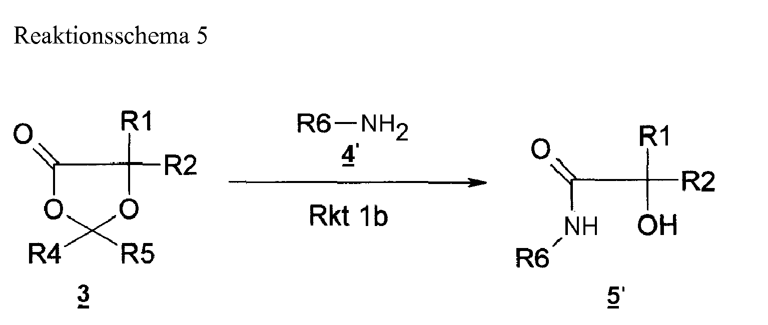

Das Reaktionsschema 6 für die Reaktion 2 mit der Verbindung der Formel **4'** R₆-NH₂ ergibt sich dann wie folgt: 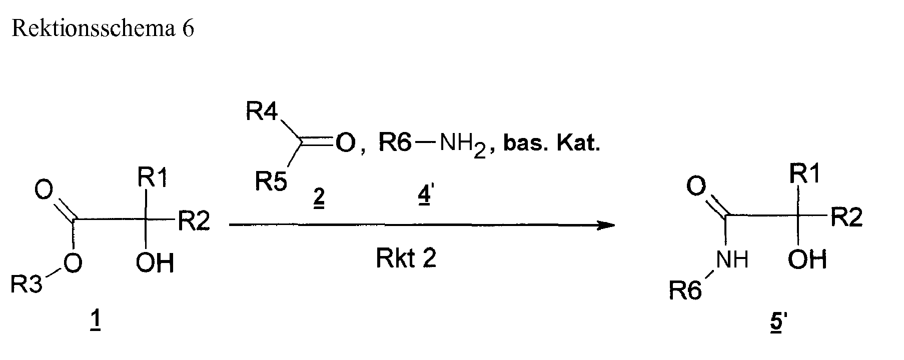 Die Reste R₁ bis R₆ haben die vorstehend angegebenen Bedeutungen.

Die Umsetzung der Verbindung der Formel **1** mit der Verbindung der Formel **2** und der Verbindung der Formel **4** bzw **4'** zur Verbindung der Formel **5** bzw. **5'** kann, abhängig vom gewählten Katalysator-Typ, bei leicht erhöhter Temperatur in einem Bereich von 25-50°C durchgeführt werden. Die Umsetzung läuft unter basischen Reaktionsbedingungen ab und funktioniert auch bei Raumtemperatur (ca. 23°C) und darunter. Bevorzugt erfolgt die Umsetzung unter milden Bedingungen bei einer Temperatur von etwa 30°C, besonders bevorzugt in einem Bereich von etwa 0 bis etwa 30°C. Die erhaltene Lösung wird in der Regel bis zur vollständigen Umsetzung gerührt. Nach Beendigung der Umsetzung werden die Verbindungen der Formel **5** bzw. **5'** aus der Lösung isoliert. Die erhaltenen Produkte können, falls erforderlich, durch Umkristallisation aus einem geeigneten Lösemittel gereinigt werden. Das erhaltene Produkt wird isoliert und gegebenenfalls im Vakuum getrocknet.

Das erfindungsgemäße Verfahren (Reaktion 2) verläuft selektiv, Nebenreaktionen werden weitgehend unterbunden.

Das erfindungsgemäße Verfahren (Reaktion 2) erlaubt eine Umsetzung unter sehr schonenden Bedingungen, die im Vergleich zu Reaktionen in hochpolaren aprotischen Lösemitteln weniger Nebenreaktionen eingeht. Die Verwendung von Aceton als Lösemittel bietet einen ganz besonderen Vorteil, da die Affinität (intramolekulare Wechselwirkung) des im chemischen Gleichgewicht gebildeten Methanols im Vergleich zu anderen Lösemittel, wie DMF oder NMP, sehr niedrig ist. So wird beim Mischen in Aceton Endothermie beobachtet, während Exothermie beim Mischen von Methanol in DMF auftritt. Es wird eine deutlich verbesserte Ausbeute gegenüber den bekannten Verfahren erhalten. Die Umsetzung kann beispielsweise auch zur Synthese empfindlicher säurelabiler und/oder temperaturempfindlicher Systeme herangezogen werden.

Besonders bevorzugt werden durch das erfindungsgemäße Verfahren (Reaktion 1b, Reaktion 1a + Reaktion 1b oder Reaktion 2) 2-Hydroxycarbonsäureester, besonders bevorzugt 2-Hydroxycarbonsäuremethylester, oder 2-Hydroxycarbonsäureamide hergestellt. Diese können unter Beibehaltung der beschriebenen basischen Reaktionsbedingungen ohne Isolierung der Zwischenstufe in einem Schritt. (Reaktion 1a + Reaktion 1b = Reaktion 2) erhalten werden.

Von besonderem Vorteil hat sich das erfindungegmäße Umesterungsverfahren (Reaktion 2) erwiesen, wenn die Verbindung der Formel **4** ein Alkohol ist, der als quaternäres Ammoniumsalz der Hexafluorophosphorsäure zum Einsatz kommen kann, weil diese Salze in Aceton löslich sind. Beispielsweise gelingt so die Herstellung von Tiotropium-Verbindungen auf sehr einfach Weise unter guten Ausbeuten.

Ein weiterer Aspekt der vorliegenden Erfindung betrifft daher ein Verfahren zur Herstellung von Tiotropiumsalzen der Formel **6** 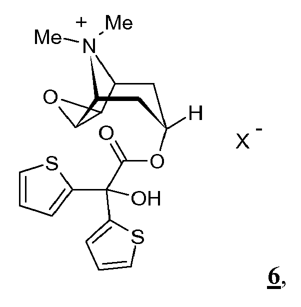 worin
- X -: ein einfach negativ geladenes Anion, vorzugsweise ein Anion, ausgewählt aus der Gruppe, bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat, p-Toluolsulfonat und Trifluormethansulfonat, bedeuten kann,
wobei eine Verbindung der Formel **1** 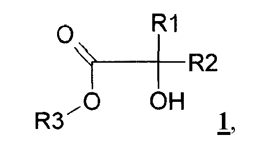 worin
- R₁ und R₂: beide Thienyl bedeuten und
- R₃: einen Rest, ausgewählt aus der Gruppe, bestehend aus Methyl, Ethyl, Propoyl, Isopropyl, Isopropenyl, Butyl, -N-Succinimid, -N-Phtalimid, Phenyl, Nitrophenyl, Fluorophenyl, Pentafluorophenyl, Vinyl, 2-Allyl bedeutet,
in einem Schritt mit einer Verbindung der Formel R₆-OH mit der chemischen Formel **7** 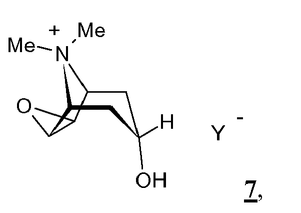 worin
- Y⁻: Hexafluorophosphat darstellt,
mit einer Verbindung der Formel **2** , vorzugsweise aus Aceton unter Zusatz eines basischen Katalysators in Form eines tert.-Alkoholats in Gegenwart von Zeolith zu einer Verbindung der Formel **8** 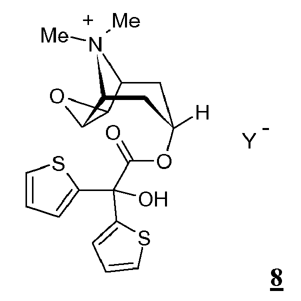 umgesetzt wird, wobei die Gruppe Y⁻ die vorstehend genannte Bedeutung aufweist, und die Verbindung der Formel **8** ohne Isolierung durch Umsetzung mit einem Salz Kat⁺X⁻, wobei Kat⁺ für ein Kation, ausgewählt aus der Gruppe, bestehend aus Li⁺, Na⁺, K⁺, Mg²⁺, Ca²⁺, organische Kationen mit quartärem N (z.B. N,N-Dialkylimidazolium, Tetraalkylammonium ) steht, und X⁻ die vorstehend genannten Bedeutungen haben kann, in die Verbindung der Formel **6** überführt wird.

Von besonderem Vorteil hat es sich bei der Aufarbeitung der hergestellten 2-Hydroxycarbonsäureester gezeigt, dass Ester, die gleichzeitig quaternäre Ammoniumsalze von Hexafluorophosphaten sind, unter Verwendung von Lithiumsalzen in andere Salze überführt werden können. Hierdurch können die Produkte beispielsweise über eine Fällungskristallisation isoliert werden und können dann gegebenenfalls in einem geeigneten Lösemittel umkristallisiert werden.

Unter quaternären Ammoniumsalzen von Hexafluorophosphaten werden erfindungsgemäß Verbindungen verstanden, welche nachfolgend durch die allgemeine Formel **9** dargestellt sind: 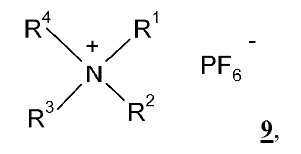 worin R¹, R², R³ und R⁴ die entsprechenden organischen Reste darstellen. Diese werden beispielsweise durch Umsetzen von Ammoniumsalzen der nachfolgenden allgemeinen Formel **9'**: 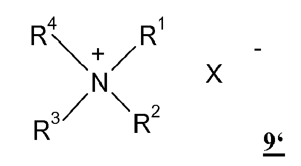 in einem geeigneten Lösemittel mit einem Salz Kat⁺PF⁶⁻ hergestellt, wobei Kat⁺ für ein Kation, ausgewählt aus der Gruppe, bestehend aus Li⁺, Na⁺, K⁺, Mg²⁺, Ca²⁺, steht.

Die Salze quaternärer Ammoniumverbindungen , wie beispielsweise diejenigen der Formel **9',** sind im Allgemeinen sehr gut wasser- und alkohollöslich. Sie sind jedoch ausgesprochen schwer löslich in weniger polaren organischen Solventien, wie beispielsweise Aceton, Acetonitril, Kohlenwasserstoffen, Halogenkohlenwasserstoffen oder Ethern. Die chemische Umsetzungen mit quaternären Ammoniumverbindungen wäre deshalb im vorliegenden Fall auf Umsetzungen in Wasser, Alkohol oder stark polare aprotische Lösemittel, wie DMF (Dimethylformamid) oder NMP (N-Methylpyrrolidin) beschränkt. Durch Umwandlung der quaternären Ammoniumverbindungen in die entsprechenden Hexafluorophosphate der Formel **9** können jedoch weniger polare aprotische Lösemittel, wie zum Beispiel Aceton, zum Einsatz kommen.

Nach erfolgter Umsetzung der Hexafluorophosphate zu den gewünschten modifizierten Ammoniumhexafluorophosphaten, lässt sich unter Verwendung von beispielsweise Lithiumsalzen (wie z.B. LiBr) das Hexafluorophosphat durch andere Anionen wieder ersetzen.

Die Vorteile der Erfindung sind vielschichtig:

Das erfindungsgemäße Verfahren (Reaktion 1a) erlaubt eine Umsetzung unter sehr schonenden basischen Bedingungen, die im Vergleich zu Reaktionen in hochpolaren aprotischen Lösemitteln weniger Nebenreaktionen und höhere Ausbeute bereitstellt. Eine Synthese von säure- und/oder temperaturempfindlichen Verbindungen ist möglich.

Erfindungsgemäß kann in einem Schritt (Reaktion 2) eine Umesterung durchgeführt werden. Von besonderem Vorteil hat es sich herausgestellt, wenn als Reaktionsmedium eine Kombination Aceton/tert.-Alkoholat bzw. Aceton/Acetonitril/tert.-Alkoholat für die erfindungsgemäße Umesterungsreaktion eingesetzt wird. Durch Gegenwart von Zeolith werden außerdem die üblicherweise auftretenden Selbstkondensationsreaktionen des Acetons oder des Acetonitrils unterdrückt. Durch die Verwendung von Zeolithe wird ferner der freiwerdende Alkohol dem Gleichgewicht effektiv entzogen.

Da es eine große Anzahl pharmakologisch wirksamer 2-Hydroxycarbonsäureester gibt, ist das erfindungsgemäß einfache Herstellungsverfahren für diese Verbindungen, sowie für deren mögliche Vorstufen in Form des 1,3-Dioxolan-2-ons besonders vorteilhaft, zumal die Beschränkung auf saure Katalysebedingungen entfällt und die Synthesebedingungen sehr sanft sind.

Neben den 2-Hydroxycarbonsäureestern können erfindungsgemäß auch die 2-Hydroxycarbonsäureamide hergestellt werden.

Erfindungsgemäß wurden zudem 1,3-Dioxolan-2-one des Typs B als Anticholinergika identifiziert. Insbesondere stellen die Verbindungen mit der Formel **610** hoch potente anticholinerge Wirkstoffe dar. Weiterhin können erfindungsgemäß herstellbare 1,3-Dioxolan-2-on-Verbindungen als Ausgangmaterialien zur Herstellung von pharmakologisch aktiven Carbonsäureestern, wie z.B. Tiotropiumbromid, dienen. Dies kann beispielsweise aus/über Verbindungen vom oben beschriebenen Typ A erfolgen.

Die nachfolgenden Beispiele dienen der Illustration exemplarisch durchgeführter Syntheseverfahren. Sie sind lediglich als mögliche, exemplarisch dargestellte Vorgehensweisen zu verstehen, ohne die Erfindung auf deren Inhalt zu beschränken.

### Beispiele

### Synthesebeispiele zur Reaktion 1a

### Allgemeine Arbeitsvorschrift:

Der 2-Hydroxycarbonsäureester (30mMol) wurde in einem Überschuß des entsprechenden Ketones/Aldehyds gelöst, dann mit einem Zeolithen des Typs Na₁₂Al₁₂Si₁₂O₃₆ x n H₂O (Porengröße 4 Ä) (6-24 g) und einer katalytischen Menge eines tert.-Alkoholats versetzt und bei 20-23°C bis zum Abschluß der Reaktion gerührt.

In Analogie zu vorstehender allgemeiner Arbeitsvorschrift wurden die folgenden Verbindungen erhalten:

| | **Bsp.1** | **Bsp.2** | **Bsp.3** | **Bsp.4** | **Bsp.5** | **Bsp.6** | **Bsp.7** | **Bsp.8** | **Bsp.9** | **Bsp.10** |
|---|---|---|---|---|---|---|---|---|---|---|
| R₁ | H | Ph | 2-Thienyl | Ph | Ph | Ph | Ph | Ph | Me | Me |
| R₂ | Ph | Ph | 2-Thienyl | Ph | H | Ph | Ph | Ph | Me | Me |
| R₃ | Me | Me | Me | Me | Me | Me | Me | Me | Me | Me |
| R₄ | Me | Me | Me | -(CH₂)₅- | -(CH₂)₅- | Isopropyl | Ph | Ph | -(CH₂)₅- | Ph |
| R₅ | Me | Me | Me | Ring | Ring | H | Ph | H | Ring | H |
| Ausbeute | 45% | 70,3 | 29,3 | 67,6 | 67,2 | 40,5 | 0 | 0 | 99 | 22 |
| Fp. | 46- 47°C | 38- 40°C | 66-68°C | Öl | Öl | Öl | - | - | Öl, 80% Gehalt | Öl |

- In Beispiel 7 wurde THF als Lösemittel verwendet.
- In Beispiel 9 wurde Cyclohexanon in equimolarem Verhältnis eingesetzt.
- In den Beispielen 1-4 und 6 wurde das Produkt über präparative Chromatographie mit Cyclohexan/Essigester = 9:1 gereinigt.
- In Beispiel 2 wurden Kristalle aus Wasser erhalten.
- In Beispiel 1 und 3 wurden Kristalle aus Cyclohexan erhalten.

### Synthesebeispiel:

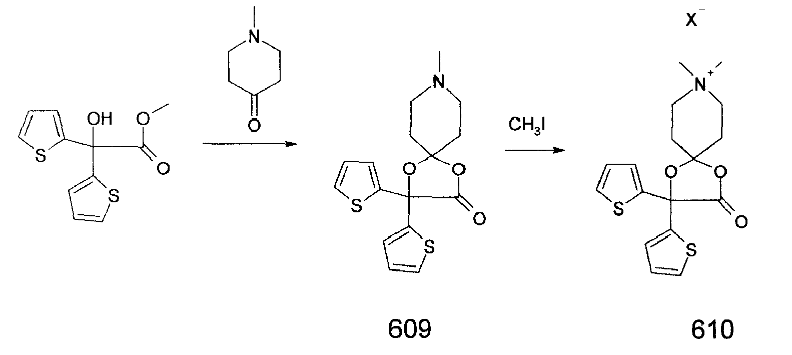

3g (12mMol) Dithienylglycolsäuremethylester, 5 g (12 mMol) N-Methyl-4-piperidon, 3 g Zeolith des Typs Na₁₂Al₁₂Si₁₂O₃₆ x n H₂O und 20 mg KOtBu werden zusammengemischt. Nach 15 h bei RT werden 200 ml Toluol zugegeben, der Zeolith abfiltriert, das Filtrat 3 x mit 500 ml Wasser gewaschen und und 2x mit 50ml verdünnter Salzsäure extrahiert. Die wässerigen Extrakte werden mit KHCO₃-Lösung versetzt und mit 200 ml Toluol gegenextrahiert. Nach Einengen wird aus Diisopropylether kristallisiert, gewaschen und getrocknet. Ausbeute: 1,3 g (Fp: 102-103°C) der Verbindung 609.

1,1g (3,2 mMol) der Verbindung 609 werden in 30 ml Acetonitril gelöst und mit 1,5 mol-Eq. (5 mMol) Methyliodid versetzt. Nach 3 h wird mit 50 ml Ether versetzt, abfiltriert, mit Ether gewaschen und getrocknet. Ausbeute : 1,5g (Fp.: 229-230°C)

### Synthesebeispiele zur Reaktion 2

Umesterung ohne Isolierung des Zwischenprodukts mit direkter Isolierung des Esters

### Allgemeine Arbeitsvorschrift:

Eine acetonische Lösung aus 0,12 mol Carbonsäureester und 0,1 mol Alkohol wird mit 90g eines Zeolithen des Typs Na₁₂Al₁₂Si₁₂O₃₆ x n H₂O (Typ 4A) und 1mmol eines tertiären Alkoholats versetzt und bei Temperaturen im Bereich 0-30°C gerührt. Nach Erreichen des chemischen Gleichgewichts wird der feste Anteil abfiltriert und das Filtrat aufgearbeitet.

Für Ester, die gleichzeitig quaternäre Ammoniumsalze von Hexafluorophosphaten sind, wird das Bromid durch Zugabe einer Lithiumbromid-Lösung (8,7g LiBr in 100ml Aceton) über eine Fällungskristallisation isoliert und anschließend aus Methanol umkristallisiert.

### Synthesebeispiele:

### Beispiel 1

### Herstellung von Hydroxy-thiophen-2-yl-thiophen-3-yl-essigsäure-9-methyl-3-oxa-9-aza-tricyclo[3.3.1.0^{2,4}]non-7-yl-ester aus Scopin (9-Methyl-3-oxa-9-aza-tricyclo [3.3.1.0^{2,4}]nonan-7-ol) und Dithienylglycolsäuremethylester.

Ausbeute: 20% (HPLC-Fläche), Identitätsbestimmung durch RRT (mit Vergleichssubstanz) per HPLC
Besonderer Vorteil: Das Scopin wird unter diesen Bedingungen nicht in Scopolin umgelagert.
Durch Einleiten von Methylbromid erhält man das entsprechende quaternäre Ammoniumbromid, welches abfiltriert und aus Wasser umkristallisiert wird.
Ausbeute: 10% (nicht optimiert)

### Beispiel 2:

### Herstellung von 3-(2-Hydroxy-2-thiophen-2-yl-2-thiophen-3-yl-acetoxy)-8,8-dimethyl-8-azonia-bicyclo[3.2.1]octan als Bromid

N-Methyltropinium PF₆ und Dithienylglycolsäuremethylester werden in Aceton gelöst und entsprechend der allgemeinen Arbeitsvorschrift behandelt.
Identitätsbestimmung durch RRT (mit Vergleichssubstanz) per DC

### Beispiel 3:

### Herstellung von 7-(2-Hydroxy-2,2-diphenyl-acetoxy)-9,9-dimethyl-3-oxa-9-azonia-tricy-clo[3.3.1.0^{2,4}]nonan

Benzilsäuremethylester und N-Methylscopiniumhexafluorophosphat werden in Aceton gelöst und entsprechend der allgemeinen Arbeitsvorschrift behandelt. Nach Abschluß der Reaktion kristallisiert man das Produkt durch Zugabe einer acetonischen Lithiumbromid-Lösung aus und kristallisiert aus Methanol um.
a) Bromid: Ausbeute (nicht optimiert) 57%
   Fp.: 178-182°C, Identitätssicherung durch RRT (mit Vergleichssubstanz) per DC
b) Hexafluorophosphat: Durch Fällen mit Natriumhexafluorophosphat in Wasser aus dem Bromid: 82% , Fp.: 178-182°C , Identitätssicherung durch RRT (mit Vergleichssubstanz) per DC

### Beispiel 4:

### Herstellung von 9,9-Dimethyl-7-(9-hydroxy-9H-fluorene-9-carbonyloxy)-3-oxa-9-azonium-tricyclo[3.3.1.0^{2,4}]nonan Bromid

9-Hydroxy-fluoren-9-carbonsäuremethylester und N-Methylscopiniumhexafluorophosphat werden in Aceton gelöst und entsprechend der allgemeinen Arbeitsvorschrift behandelt. Nach Abschluß der Reaktion kristallisiert man das Produkt durch Zugabe einer acetonischen Lithiumbromid-Lösung aus und kristallisiert aus Methanol um.
a) Bromid: Ausbeute (nicht optimiert) 46% Fp.: 238-241°C
b) Hexafluorophosphat: Durch Fällen mit Natriumhexafluorophosphat in Wasser aus dem Bromid: 70 %, Fp.: 265-271°C (Z)

## Patentansprüche

1. Verfahren zur Herstellung von 1,3-Dioxolan-2-on der Formel **3** 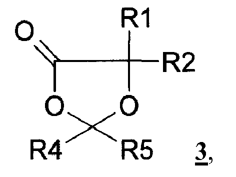 worin
R₁ und R₂ unabhängig voneinander jeweils Wasserstoff oder einen Aryl- oder Heteroaryl-Rest bedeuten, wobei der Aryl- oder Heteroaryl-Rest jeweils gegebenenfalls ein- oder mehrfach substituiert sein kann;
R₄ und R₅ jeweils unabhängig voneinander ausgewählt sind aus Wasserstoff, C₁-C₆-Alkyl, oder R₄ und R₅ zusammen einen gesättigten oder ein- oder mehrfach ungesättigten carbocyclischen oder heterocyclischen Ring, der ein oder mehrere Heteroatom, ausgewählt aus S, N oder O, enthalten kann, der gegebenenfalls jeweils unabhängig voneinander ein oder mehrfach substituiert sein kann, bilden, wobei R₄ und R₅ nicht gleichzeitig Wasserstoff sein können;
wobei ein 2-Hydroxycarbonsäureester der Formel **1** 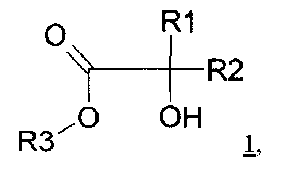 worin
R₁ und R₂ wie oben definiert sind und
R₃ Methyl, Ethyl, Propyl, Isopropyl, Isopropenyl, Butyl, N-Succinimid, N-Phtalimid, Phenyl, Nitrophenyl, Fluorophenyl, Pentafluorophenyl, Vinyl, 2-Allyl bedeutet,
in einem geeigneten Lösemittel unter Zusatz eines geeigneten basischen Katalysators in Gegenwart von Zeolith in einem Schritt mit einer Verbindung der Formel **2** 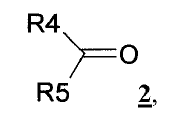 umgesetzt wird, worin R₄ und R₅ wie oben definiert sind.

2. Verfahren zur Herstellung einer Verbindung der Formel **5** 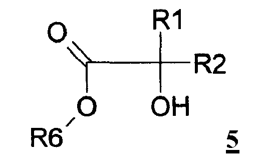 worin
R₁ und R₂ wie in Anspruch 1 definiert sind;
R₆ einen organischen Rest bedeutet,
wobei ein 2-Hydroxycarbonsäureester der Formel **1** 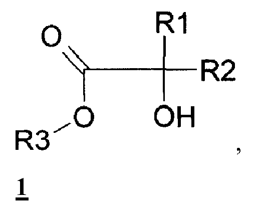 worin
R₁ und R₂ wie in Anspruch 1 definiert sind;
R₃ unterschiedlich zu R₆ ist und ausgewählt ist aus Methyl, Ethyl, Propyl, Isopropyl, Isopropenyl, Butyl, N-Succinimid, N-Phtalimid, Phenyl, Nitrophenyl, Fluorophenyl, Pentafluorophenyl, Vinyl, 2-Allyl,
in einem ersten Schritt in einem geeigneten Lösemittel unter Zusatz eines geeigneten basischen Katalysators in Gegenwart von Zeolith in einem Schritt mit einer Verbindung der Formel **2** 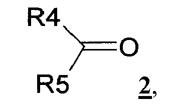 worin R₄ und R₅ wie oben definiert sind,
zu einer Verbindung der Formel **3** 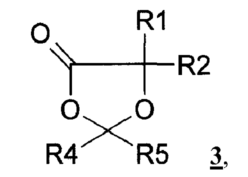 worin R₁, R₂, R₄ und R₅ die vorstehend genannten Bedeutungen haben, umgesetzt wird, und worin
die Verbindung der Formel **3** in einem zweiten Schritt in einem geeigneten Lösemittel unter Zusatz eines geeigneten basischen Katalysators, in Gegenwart von Zeolith mit einer Verbindung der Formel **4**
**R₆-O-H** 4
worin
R₆ einen organischen Rest bedeutet, der von R₃ unterschiedlich ist,
zu einer Verbindung der Formel **5** umgesetzt wird.

3. Verfahren zur Herstellung einer Verbindung der Formel **5** 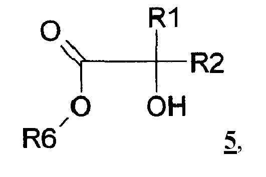 worin
R₁ und R₂ wie in Anspruch 1 definiert sind und
R₆ wie in Anspruch 2 definiert ist;
wobei ein 2-Hydroxycarbonsäureester der Formel **1** 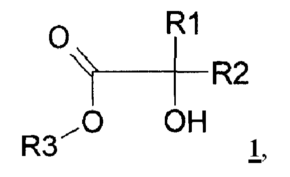 worin
R₁ und R₂ und R₃ wie in Anspruch 1 definiert sind;
in einem Schritt mit einer Verbindung der Formel **2,** 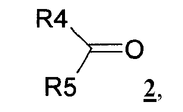 worin R₄ und R₅ wie oben definiert sind und einer Verbindung der Formel **4**
**R₆-O-H** 4
worin
R₆ einen organischen Rest bedeutet, der von R₃ unterschiedlich ist,
in einem geeigneten Lösemittel unter Zusatz eines geeigneten basischen Katalysators in Gegenwart von Zeolith zu einer Verbindung der Formel **5** umgesetzt wird.

4. Verfahren nach Anspruch 2 oder 3, **dadurch gekennzeichnet, dass** statt der Verbindung R₆-OH eine Verbindung R₆-NH₂ ausgewählt wird, um ein 2-Hydroxycarbonsäureamid herzustellen.

5. Verfahren nach mindestens einem der vorangehenden Ansprüche 1 bis 4, **dadurch gekennzeichnet, dass** die Umsetzung bei einer Temperatur in einem Bereich von etwa 0 bis etwa 30°C durchgeführt wird.

6. Verfahren nach mindestens einem der vorangehenden Ansprüche 1 bis 5, **dadurch gekennzeichnet, dass** das tert. Alkoholat ausgewählt wird aus Alkali- oder Erdalkalialkoholaten, bevorzugt Natrium- oder Kalium-tert.-butylat oder Natrium- oder Kalium-tert.-amylat.

7. Verfahren nach mindestens einem der vorangehenden Ansprüche 1 bis 6, **dadurch gekennzeichnet, dass** der Zeolith ausgewählt wird aus Alkali- oder Erdalkalihaltigen Alumosilikaten, bevorzugt Natrium- oder Kalium-haltigen Alumosilikaten, insbesondere Molekularsieben mit der Summenformel Na₁₂[(AlO₂)₁₂(SiO₂)₁₂] x H₂O oder Na₁₂Al₁₂Si₁₂O₃₆ x n H₂O, wobei n bevorzugt < 6 (Molekularsieb Typ 4A) erfindungsgemäß besonders bevorzugt ist.

8. Verfahren nach mindestens einem der vorangehenden Ansprüche 1 bis 7, **dadurch gekennzeichnet, dass** als Lösemittel Ketone, Nitrile, oder Heteroaromaten, , bevorzugt Aceton ,Pyridin, und Acetonitril verwendet werden.

9. Verfahren nach mindestens einem der vorangehenden Ansprüche 1 bis 8, **dadurch gekennzeichnet, dass** R₃ ausgewählt wird aus Methyl, Ethyl, Propyl und Butyl, besonders bevorzugt Methyl oder Ethyl.

10. Verfahren nach mindestens einem der vorangehenden Ansprüche 1 bis 9, **dadurch gekennzeichnet, dass** R₁ und R₂ jeweils ausgewählt werden aus einem Heteroaryl-Rest jeweils gegebenenfalls ein- oder mehrfach substituiert mit Halogenatomen, wie Fluor, Chlor, Brom oder Jod, -CN, C₁-C₄-Alkyl, C₁-C₄-Alkoxy, Hydroxy, oder CF₃.

11. Verfahren nach mindestens einem der vorangehenden Ansprüche 1 bis 10, **dadurch gekennzeichnet, dass** für R₁ und R₂ derselbe Heteroaryl-Rest eingesetzt wird.

12. Verfahren nach mindestens einem der vorangehenden Ansprüche 1 bis 11, **dadurch gekennzeichnet, dass** für R₁ und R₂ jeweils der Thienyl-Rest eingesetzt wird.

13. Verfahren nach mindestens einem der vorangehenden Ansprüche 1 bis 12, **dadurch gekennzeichnet, dass** R₄ und R₅ unabhängig ausgewählt werden aus C₁-C₆-Alkyl, besonders bevorzugt Methyl und Ethyl, ganz besonders bevorzugt sind R₄ und R₅ beide Methyl.

14. Verfahren nach mindestens einem der vorangehenden Ansprüche 1 bis 13, **dadurch gekennzeichnet, dass** R₄ und R₅ zusammen einen gesättigten carbocyclischen oder heterocyclischen Ring bilden, der jeweils gegebenenfalls ein oder mehrfach substituiert ist mit Halogenatomen, wie Fluor, Chlor, Brom oder Jod, -CN, C₁-C₄-Alkyl, C₁-C₄-Alkoxy, Hydroxy, CF₃ und -O-COR', wobei R' einen Rest darstellt, ausgewählt aus der Gruppe, bestehend aus C₁-C₄-Alkyl, Benzyl und Phenylethyl, der jeweils substituiert sein kann durch Hydroxy, Hydroxymethyl oder Methoxy, besonders bevorzugt R₄ und R₅ zusammen einen Pyridinyl-Ring bilden.

15. Verfahren nach mindestens einem der vorangehenden Ansprüche 1 bis 14, **dadurch gekennzeichnet, dass** R₄ und R₅ gemeinsam mit einem Stickstoffatom einen heterocyclischen Ring bilden, ausgewählt aus Pyrrol, Pyrrolin, Pyrrolidin, Pyridin, Piperidin, Morpholin, 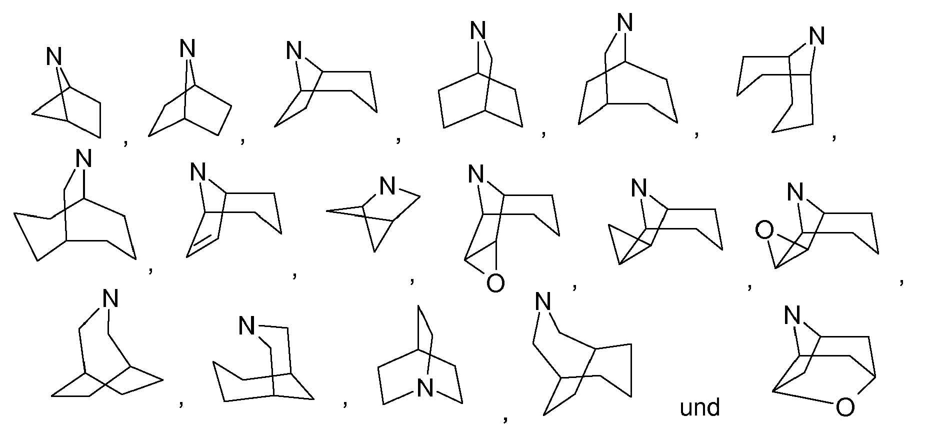 der gegebenenfalls substituiert sein kann durch einen oder mehrere Reste, bevorzugt einen Rest, ausgewählt aus der Gruppe, bestehend aus OH, F, Methyl, Ethyl, Methoxy und -O-COR', wobei R' einen Rest darstellt, ausgewählt aus der Gruppe, bestehend aus C₁-C₄-Alkyl, Benzyl und Phenylethyl, der jeweils substituiert sein kann durch Hydroxy, Hydroxymethyl oder Methoxy.

16. Verfahren nach Anspruch 2, **dadurch gekennzeichnet, dass** die Umsetzung ohne Isolierung oder mit Isolierung der Zwischenstufe der Verbindung der Formel **3** durchgeführt wird.

17. Verfahren nach Anspruch 2 oder 3, **dadurch gekennzeichnet, dass** die Verbindung der Formel **4** in Form eines quaternären Ammoniumsalzes der Hexafluorophosphorsäure eingesetzt wird.

18. Verfahren nach einem der Ansprüche 1 bis 3, **dadurch gekennzeichnet, dass** als Ausgangsmaterial und/oder als Endprodukt ein säureempfindlicher 2-Hydroxycarbonsäureester vorliegt.

19. Verfahren zur Herstellung von Tiotropiumsalzen der Formel 6 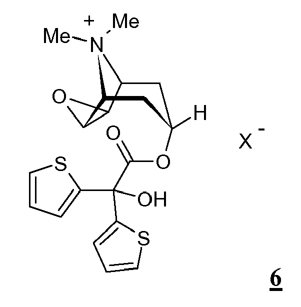 worin
X - ein einfach negativ geladenes Anion, vorzugsweise ein Anion, ausgewählt aus der Gruppe, bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat, p-Toluolsulfonat und Trifluormethansulfonat, bedeuten kann,
wobei eine Verbindung der Formel **1** 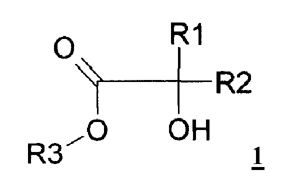 worin
R₁ und R₂ beide 2-Thienyl bedeuten und
R₃ einen Rest, ausgewählt aus der Gruppe, bestehend aus Methyl, Ethyl, Propoyl, Isopropyl, Isopropenyl, Butyl, -N-Succinimid, -N-Phtalimid, Phenyl, Nitrophenyl, Fluorophenyl, Pentafluorophenyl, Vinyl, 2-Allyl bedeutet,
in einem Schritt mit einer Verbindung der Formel **7** 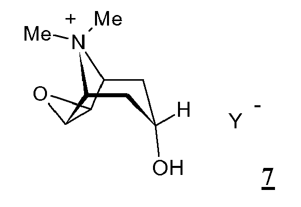 worin
Y⁻ Hexafluorophosphat darstellt,
mit einer Verbindung der Formel **2** , vorzugsweise Aceton, unter Zusatz eines basischen Katalysators in Form eines tert.-Alkoholats in Gegenwart von Zeolith zu einer Verbindung der Formel **8** 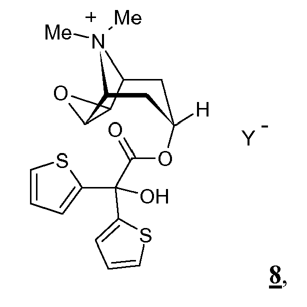 umgesetzt wird, wobei die Gruppe Y⁻ die vorstehend genannte Bedeutung aufweist, und die Verbindung der Formel **8** ohne Isolierung durch Umsetzung mit einem Salz Kat⁺X⁻, wobei Kat⁺ für ein Kation ausgewählt aus der Gruppe bestehend aus Li⁺, Na⁺, K⁺, Mg²⁺, Ca²⁺, organische Kationen mit quartärem N (z.B. N,N-Dialkylimidazolium, Tetraalkylammonium ) steht, und X⁻ die vorstehend genannten Bedeutungen haben kann, in die Verbindung der Formel **6** überführt wird.

20. Verfahren nach mindestens einem der vorangehenden Ansprüche 1 bis 19, **dadurch gekennzeichnet, dass** ein 2-Hydroxycarbonsäureester der Formel **5**, der in Form eines quaternären Ammoniumsalzes von Hexafluorophosphat erhalten wird, durch Zugabe eines Lithiumsalzes in einem aprotischen Lösemittel, bevorzugt Aceton, über eine Fällungskristallisation aus dem Reaktionsmedium isoliert werden kann.

21. 1,3-Dioxolan-2-on mit der Grundstruktur vom Typ A und 1,3-Dioxolan-2-on mit der Grundstruktur vom Typ B:

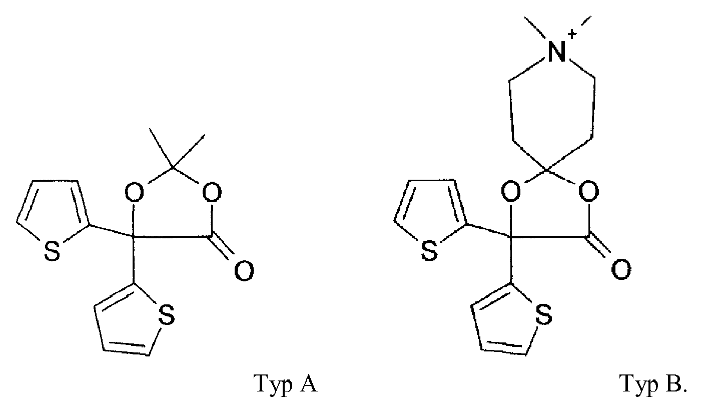

22. Verwendung von 1,3-Dioxolan-2-on mit der Grundstruktur vom Typ B nach Anspruch 21 als anticholinerger Wirkstoff.

23. Verwendung von 1,3-Dioxolan-2-on mit der Grundstruktur vom Typ B nach Anspruch 21 zur Herstellung eines Arzneimittels zur Behandlung von Asthma, COPD oder anderen Krankheiten, die sich durch Inhibierung muskarinischer Rezeptoren behandeln lassen.

24. Verbindung der Formel **610** 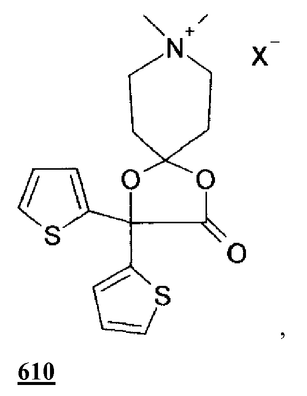 wobei X⁻ Chlorid, Bromid, Jodid, Sulfat, Phosphat, Methansulfonat, Nitrat, Maleat, Acetat, Citrat, Fumarat, Tartrat, Oxalat, Succinat, Benzoat oder p-Toluolsulfonat darstellt, wobei die Substituenten am Stickstoff ausgewählt sind aus Alkyl, bevorzugt Methyl, Ethyl,.n-Propyl, Isopropyl und die Thienyl-Reste gegebenenfalls ein oder mehrere Substituenten aufweisen, ausgewählt aus Halogenatomen, wie Fluor, Chlor, Brom oder Jod, -CN, C₁-C₄-Alkyl, C₁-C₄-Alkoxy, Hydroxy oder CF₃.

25. Verwendung der Verbindung der Formel **610** nach Anspruch 24 als anticholinerger Wirkstoff.

26. Verwendung der Verbindung der Formel **610** nach Anspruch 24 zur Herstellung eines Arzneimittels zur Behandlung von Asthma, COPD. oder anderen Krankheiten, die sich durch Inhibierung muskarinischer Rezeptoren behandeln lassen.

27. Verwendung von tert.-Alkoholat mit Zeolith Typ 4A als Umesterungskatalysator für die Veresterungen von Scopin der Formel **7.**
